# EUROPEAN PATENT SPECIFICATION

(11) **EP 1 494 740 B1**
(45) Date of publication and mention of the grant of the patent: **30.12.2009**
(21) Application number: 03717859.7
(22) Date of filing: 11.04.2003
(51) Int. Cl.: A61M 5/31

(54) **A SYSTEM FOR MODIFICATION OF A DEVICE AND A DEVICE SUITABLE FOR MODIFICATION**
SYSTEM ZUR MODIFIZIERUNG EINER VORRICHTUNG UND EINE FÜR DIE MODIFIZIERUNG GEEIGNETE VORRICHTUNG
SYSTEME DE MODIFICATION D'UN DISPOSITIF ET DISPOSITIF APPROPRIE A CETTE MODIFICATION

(30) Priority: 16.04.2002 SE 0201142
(43) Date of publication of application: 12.01.2005
(73) Proprietor: Pfizer Health AB, 112 87 Stockholm (SE)
(72) Inventor: HJERTMAN, Birger, S-162 43 Vällingby (SE); HIMBERT, Hans, S-168 56 Bromma (SE); PAVLU, Bohdan, S-131 41 Nacka (SE); GENETAY, Kerstin, S-178 93 Drottningholm (SE)
(74) Representative: Hadjadj, Laurent
(86) International application number: PCT/SE2003/000588
(87) International publication number: WO 2003/086511

(56) References cited:
- WO-A1-98/43690
- WO-A1-98/55168
- US-A- 5 520 658
- US-B1- 6 338 200

## Description

Present invention is directed to a system for modification of an injection device according to the preamble of claim 1.

Existing portable multidose apparatuses or injection devices, eg. pen-type injectors, generally consists of a first end portion with an ejection outlet, e.g. a mounting for a needle assembly, a second, opposite end portion possibly having a dosing button, and an intermediate portion serving as a handle. Said portions are integrated in a common shell-type housing of non-flexible resin, preferably an injection moulded casing. The design of the casing is adapted to protect the injector from damages when being dropped on a floor or when being subjected to bending or twisting forces, as the hard casing resists the contemplated applied forces and shelters the mechanism of the injector. However, all pen-type injectors of the same brand are identical resulting in confusion which injector belongs to who when two or more injectors at the same time and for some reason are present in the same location. Further, different individuals may need functional adaptations to there specific requirements, e.g. in respect of added feature or adapted size and shape for gripping parts. PCT application WO 98/55168 is directed to a manually operated injection apparatus having an injection moulded housing with an intermediate handle portion. A soft-touch semi circular layer is firmly attached to a part of the handle portion by for example a two-shot moulding or co-injection moulding process. This means that the injection apparatus is obtainable with a permanently fixed grip of a soft-touch semi circular, in cross section, layer. However, this grip is of a standardized design and cannot be adapted to a users personal requirements or needs. Further, this document concerns a container of medication mounted to said housing, an outlet in flow communication with said container to receive medication forced therefrom and a drive assembly adapted to interact with said container to deliver medication from the container through the outlet. Further, every injection apparatus of the same brand looks alike and are hard to distinguish from each other.

WO 98/43690 is directed to a continuous injecting apparatus comprising a hollow housing with a syringe mounted therein and a connector tightly threaded onto the front end portion of the housing and including a needle fitting portion onto which a needle is positioned. It also concerns an adjusting portion including an adaptor coupled to the rear end of the hollow housing, a plug inserted into the adaptor, a plunger including a gripper and a grasping portion formed to force a user to push down the piston of the syringe and a stopper movably mounted onto a rod of the plunger, thereby enabling the adjustment of an amount of medical liquid to be injected for diagnostic or therapeutic purposes. Furthermore, there is a marking means mounted on the side surface and handle portion of the housing to determine which domestic animal is injected or innoculated.

US Patent 5,520,658 relates to a dispensing device for dispensing at least 2 fluids and comprises a housing of a pistol-like configuration having an integral handle, in which a spring-biased trigger is received. Within the housing of the dispensing device, two syringes are received and fixated, which syringes contain respective fluids to be accurately dispensed.

It is an object of present invention to provide an element for a manually operated injection device, which is customized to the user's personal requirements and wishes.

It is another object of present invention to provide an element for an injection device holding information concerning the owner of the device, dose and kind of medical drug to be delivered to the owner in case of emergency and other vital information.

A further object of present invention is to provide an element for an injection device in which a metering device is integrated.

These and other objects to be evident from the description below are met by the characteristics set forth in the appended claims.

Known in the art are various consumer articles, e.g. cell phones etc., marketed with a plurality of add-on parts allowing the article to be personalized or customized. Typically.such add-ons only serve an aesthetical but not a functional purpose. Furthermore, faulty handling, misuse, unauthorized access or inadvertent mix-up of different individuals articles does not cause harm. In contrast, manually operated injectors may bring severe harm if not properly handled before, during and after injection. A patient identifying the wrong device is an obvious risk factor. Preparatory steps may include for example needle attachment, mixing, dose setting and deaeration of the preparation, arming of the device etc. Critical steps in the mere injection phase can be steady abutment to or penetration of the proper target site, penetration to proper depth, possibly aspiration of body fluid, delivery of full dose and withdrawal without residue. After the treatment it may be necessary to remove a contaminated needle, clean and reset the device, replace a preparation ampoule or store the device with ampoule in a refrigerator. All such steps may cause harm, e.g. by over or under dosing, destruction of tissue, injection of air, transmittal of blood borne infections or deactivation of drug. Manually operated injectors must be designed for worst-case situations including self-administration also by children, elderly and disabled persons, even when traumatized by the injection procedure itself. Optimization of a design for all possible patient and assistant groups is difficult, however. There are individual variations in hand sizes, gripping patterns, body postures and administration regimens. Patients dependent on daily administrations also have a legitimate right for consideration to individual variations in taste and preferences, e.g. for devices that need to be brought around in daily life.

The present invention serves to solve the above-indicated problems in connection with injection devices. The invention provides a system for modification of injector devices allowing variation of device properties within broad ranges. The invention offers a plurality of elements that can be attached to an injector housing. With elements having different properties a multitude of variations becomes possible for the housing part of the device, which shall be illustrated and exemplified in the detailed description below. Device size and/or shape can be affected. For example, the size of the device can be adapted to the size of individual hands, even continuously as for a growing child, or to gripping habit variations such as when using precision grip, as when using a pen, or force grip, as when using the hole fist. Even adaptations to individual normal, disabled or injured hand shapes become possible. Soft or sensitive materials can be used as a worn or destroyed element can be replaced. Elements used e.g. at gripping areas with textured surfaces can be removed and cleaned or sterilized separately even when the injector as such does not stand such treatment. Individual shapes can be used e.g. to allow identification also by a blind patient. Size and shape variations are possible also when the housing has a specific site for the element, e.g. with locking or guiding structures, as the element can extend beyond the site to varying degrees. The element can also be used to carry information of general or individual character and in human or machine readable form, the latter e.g. for security or physician verification. Aesthetic variations of individual taste can also be said to carry information. The element can also be used to add a functional property as in case of metering devices. For example, an element including a thermometer function, e.g. including liquid crystals, may help a patient verifying that the device is stored under suitable cold conditions, that it is not or has not been exposed to drug destroying temperatures or that the device has regained suitable temperature for injection. Further examples will be given below.

As indicated, a manually operated injection device can be said to comprise a) a housing designed or suitable for manual gripping, b) a container for a preparation attached to or enclosed in the housing, c) an outlet for the preparation exposed with respect to the housing, d) optionally a cap or guard arranged for removable attachment over the outlet and e) a mechanism arranged for moving the preparation at least from the container through the outlet. For description purposes the housing a) shall be regarded as providing an "encasing" function whereas the other elements b), c), d) and e) provides "operative" functions, not forming part of the housing.

The principles of the present invention may be used for injection devices or systems in broad terms utilizing various delivery principles. The device may be of disposable design but is preferably designed reusable. The device is manually operated in the sense that it shall at least be gripped, typically in connection with an injection step, but may also require additional handling steps. The injector can be entirely manually operated, e.g. wherein a control button is pushed to perform injection and possibly also manipulated to set a dose etc. The device can be more or less automatic with mechanical means, such as in autoinjectors, or with electronic and motorized means. The housing shape can vary depending on internal layout but shall have a basic form suitable for gripping. It may have the elongated and even generally cylindrical form known from injection pens. Although the elements of the present system may add to housing ergonomics and convenience properties it is preferred that can be manually operated also without such elements, e.g. for minimum size in the hands of a child. The container can be any type of single use, refillable or replaceable. Syringe type containers can be used, such as ampoules, cartridges, carpoules and syringes. The outlet from the device may be an infusion channel or any conducting means such as a tube or catheter, a needle or cannula or a needle-less system based on liquid jet or a particle gun with gas propellant. As known per se the outlet can be temporarily covered by a cap or, in connection with needles, a displaceable needle-guard. The container content material shall be deliverable by use of a delivery mechanism, also referred to herein as a pump, and any material fulfilling this requirement can be used. Normally the material is a fluid and preferably a liquid, including materials behaving as liquids such as emulsions or suspensions. These observations relates to the final preparation whereas other components, notably solids, may be present before final preparation. The nature of container preparation content shall also be understood to include medical in broad terms and to embrace for example natural components and body fluids prefilled or drawn into the container although most commonly the medical is factory prepared. The device can be used in connection with medicals requiring a preparation step immediately prior to the infusion, typically a mixing of two or more components, which all may be fluid or may include a solid as when dissolving a lyophilized powder in a solvent, such as hormones or prostaglandins. The administration manner can also be varied within broad limits and may include entirely continuous infusion, continuous infusion with varying flow or intermittent infusions or injections with repeated either equal or varying doses. In portable devices the intermittent administration is common. Similarly, although injection devices may be contemplated also for a single dosing operation, generally they are designed for more than one or multiple individual doses for intermittent administration. In addition to the basic functions for delivery purposes the delivery system with preference may include other valuable features such as for initiating the container and its content and provide various checks and controls of both the container and the pump part electronics and mechanics. The mechanism for delivery of medical through the container opening should basically include at least one type of pump which may have to be selected for the special kind or container and medical used. The pump may include any kind of pressure source, such as mechanical or electrolytic pressure build-up, in the container and suitable valve means for control, which method can be used with virtually any kind of container and any kind of product, such as transdermal delivery of powder, similar delivery through liquid jets or regular tube infusion. The common syringe type container need a specialized pump system. Either the mechanism is adapted to act on complete syringes, having their own piston rods or the mechanism has a piston rod acting more or less directly on the piston of a cartridge type container, which can be made smaller and more adapted to portable devices. Also dual or multiple chamber cartridges can use similar devices for its various phases.

The system of the present invention is defined in claim 1. It comprises i.a. at least one injection device and a set of at least two elements, which elements are different in at least one respect or property. The property may be any of the above indicated shape, information or functional respects but can be any other property as well. Preferably the property is one that is significant for the user of the device and most preferably a property that is significant to the user as an individual. The existence of two, several or a plurality of different elements means that a choice can be made and that the device can be given different properties. This is not only an advantage for the user. It also represents an advantage to a manufacturer, supplier, vendor, wholesaler, retailer etc. since one and the same basic device can be modified into several varieties, which lowers costs, facilitates logistics etc. The minimum requirement on the system is that there shall be a possibility for such a modification. Devices may be delivered from the manufacturer with an attached selected element or a standard element for optional later replacement. Alternatively devices can be delivered naked for later selection and attachment, e.g. at a vendor or a physician. The attachment can be permanent, e.g. for security or safety reasons, by use of for example interlocking means. The attachment can be made releasable only by means of a tool, e.g. in the possession of a physician for replacement or administration instructions. The attachment can be arranged manually releasable, e.g. to allow element replacement to the taste or in the discretion of the user. It is clear that the three minimum components of the system need not be at the same physical location to satisfy the requirement for modification possibility although this is also a possibility, e.g. if a kit of one injector device with several alternative elements is delivered to the user. The system can also be actually assembled, e.g. in case the housing has at least two sites for the elements, occupied by different elements.

Housing and element are preferably mutually adapted for the purpose of creating at least one specific site for the elements in a set of such elements. When attached to the site the element should be localized to that site. If movable when attached to the site, the element shall have a range of movement limited to a fraction of the housing dimensions. For most purposes it is preferred that the element is immobile when attached to the site. To this end the housing and/or the element can have guiding structures. The housing may have a basic topology of irregular or double-curved areas forming natural guiding structures for correspondingly shaped elements. Special guiding structures may be added, such as a rim, recess or platform to form a specific structurally defined seat for the element. It is preferred that the site or seat parts of the housing are designed so as to allow handling of the injector also without an element attached and preferably also these parts are sealed with respect to the housing interior, by being covered or integral with the rest of the housing, in order to prevent access or entrance of contamination with or without an attached element. Similarly the elements may have guiding structures fitting to housing details. Both parts can have complementary structures keying into each other at attachment. Although it is possible that the element is fixed to the site by gluing, fusing or similar methods it is preferred that fixation takes place by mechanical means, which among others facilitates attachment, increases flexibility, allows replacement and avoids contamination. Any known principles for mechanical attachment can be used. Preferred are attachments based on shape and/or friction. The shapes of housing and element may be such that the element encircles the housing or a housing part to such an extent that locking and release prevention are created, e.g. that the element encircles a generally cylindrical or tube formed housing or housing part to more than a half circle in cross-section as for a rings, sleeves, clamps etc. Another shape-based attachment is use of special locking structures of known kind, e.g. interlocking structures, hooks and eyes, pins and grooves, protrusions and undercuts etc. Alternatively or in addition friction can be relied on, normally requiring a force as between interlocking structures or between housing and element, the latter preferably in combination with a design encircling the housing or housing part as described and preferably with a design of the element such that it has sufficient elasticity and pre-stress in its seated condition to generate a clamping force. Friction increasing structures, textures or material can be positioned on the housing site and/or preferably on the element inner surface. As indicated above the mechanical attachment can be designed for permanent fixation, tool releasable fixation or preferably manually releasable fixation.

An element can be said to have an "inner" surface adapted for attachment to the housing site and an "outer" surface exposed to the user. It is normally the inner surface that is provided with the attachment structures and features exemplified, although the whole element may contribute as in case of clamping. A "set" of elements shall be understood as a plurality of elements designed for attachment to the same type of site, i.e. having sufficiently similar inner surfaces or attachment characteristics to allow all of them be attached to the same site. Also fulfilling the requirements of a set are elements where a part can be exchanged, e.g. a translucent or transparent cover part for exchange of different sheets beneath although in most situations it is preferred that that the entire element is exchanged. It is possible that the housing has more than one site with the same kind of attachment characteristics, allowing attachment of the elements in a set to either or all such sites, e.g. two similar panels on each side of the housing or encircling element structures at different axial parts of the housing. I is certainly also possible that the housing has a second or more types of sites for a second or more sets of elements having different attachment characteristics compared to the first set, e.g. a first site type and set for information and a second or more site types and sets for gripping or any other function. Different caps or needle-guards may form one or two sets of element within the present meaning. When having more than one site type it is desirable the sets comprises elements that match between the sets in functional, ergonomic and/or aesthetical respects.

The attachment characteristics between housing and element can be designed so as to allow attachment in a movable manner, as indicated above, or at least to allow attachment of the element in more than one way, e.g. to relieve the user from considering what is left/right, front/rear etc. in case of symmetrically designed elements. In many instances it is preferred that the elements can be attached in only one fixed orientation, e.g. when dictated by function or asymmetry. The outer surface of the element may extend over substantially the same area as the inner surface or the housing site or seat but may also extend beyond such an area, e.g. for set of elements allowing variation of gripping areas, but the elements may also have the same overall shape, e.g. for other purposes. The nature of the outer surface is dependent on its function, and variations will be evident from the exemplification. The same applies for the overall shape of the element.. A preferred class of shapes is "shell" formed elements, meaning that they substantially conforms to surface area of the housing and essentially forms a continuation of the housing exterior when attached although with slight deviations such as slightly recessed or elevated or with about the same elevation, e.g. when placed in a recess in the housing so as to make the element about flush with the surrounding housing exterior. To act as a shell in this meaning the element with preference is "thin" with respect to housing dimensions, e.g. less than 6 mm, preferably less than 4 and most preferably less than 2 mm. The shell can have varying cross-section thickness over its surface but preferably the shell has about constant cross-section thickness. The element or shell may cover the entire housing although modification advantages are reached it the element only occupies a part, e.g. less than 50% and preferably less than 30% of the housing total area, for example when applied only where functionally needed or sufficient for displaying information. It is preferred that the element only.partly encircles the housing circumference. It is also preferred that the element only partly extends along the housing axis. To be further explained below, a shape found useful is a shell having substantially U-shaped cross-section and designed to clamp around slightly more than half a housing circumference and having an axial extension of less than housing length but more than the diameter of the U-shaped cross-section, preferably more than 2 and most preferably more than 3 timed the diameter.

Preferred embodiments of the invention are illustrated below with reference to the accompanying drawings, in which:
Fig. 1 in an exploded side view shows a pen-type injection device without the injection needle but exposing a needle mounting and its protecting cap, whereby two elements according to present invention are depicted below the handle section of the injection device;
Fig. 2 illustrates the injection device of Fig. 1 in an assembled state and with a grip means according to present invention having a textured surface for gripping;
Fig. 3 illustrates the injection device of Fig. 2 but with an outer face provided with an elected pattern;
Fig. 4 schematically depicts the injection device of Fig. 2 and with a grip means according to present invention having a contour matching the palm and the fingers of the user of said device;
Fig. 5 is a cross section view of an element according to present invention;
Fig. 6 is a cross section of an example element that does not fall within the scope of the invention.
Fig. 7-14 are cross section views of alternative, contemplated embodiments of an element according to present invention, wherein Fig. 10 also is a cross section view along line A-A and Fig. 11 also is a cross section view along line B-B in Fig. 4;
Fig. 15 is an elevation view of the embodiment in Fig 12; and
Fig. 16 is an exemplifying embodiment of an element according to present invention having a protruding fastening element for attaching a charm, a mascot or an anchoring chain to the injection apparatus of Fig 1.

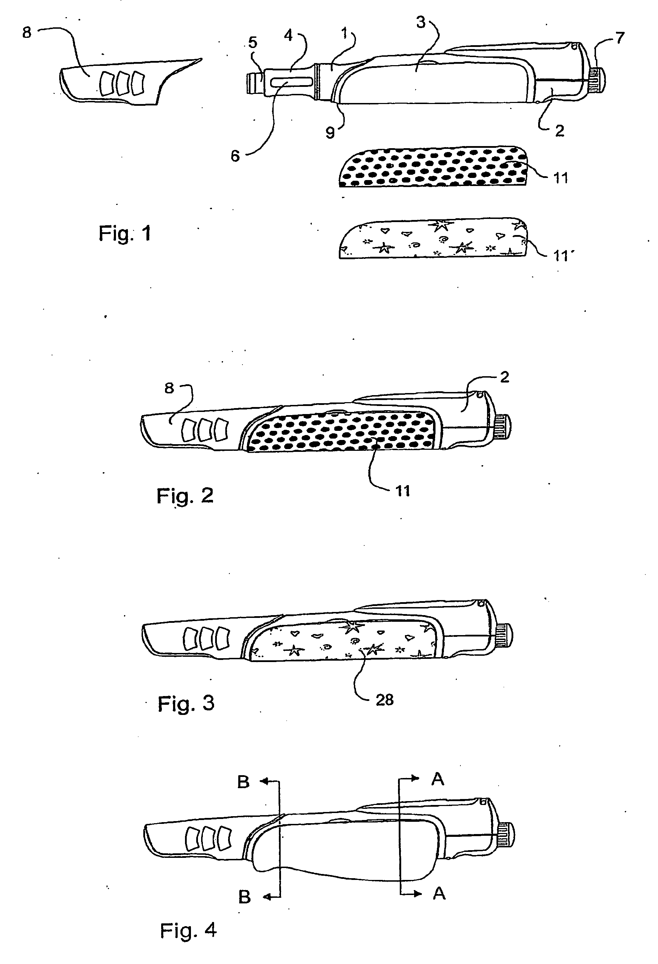

Referring first to Fig. 1 illustrating a portable multidose device in the form of a pen-type injector, said device generally comprises an injection moulded casing or housing consisting of a first end portion 1 and a second end portion 2. A handle section 3 or an area part of the housing serving as a handle is integrated in the second end portion between the ends of the casing. An injection outlet 4 is attached to the outermost end of the first end portion 1. In the illustrated embodiment the injection outlet is provided with a mounting (external threads) 5 for fastening of an injection needle (not shown) and has a cavity 6 adapted to receive an ampoule or container (not shown) holding a drug or a medical preparation to be self-injected by the owner of said injection apparatus. A mechanism for injecting a dose of a drug is housed inside said casing and Fig. 1 depicts a button 7 at the opposite end of the casing serving both as a dosing device and a trigger for the injection action. An optional protecting cap 8 is removably attached to said first end portion and protects the injection outlet when the injection device not is used.

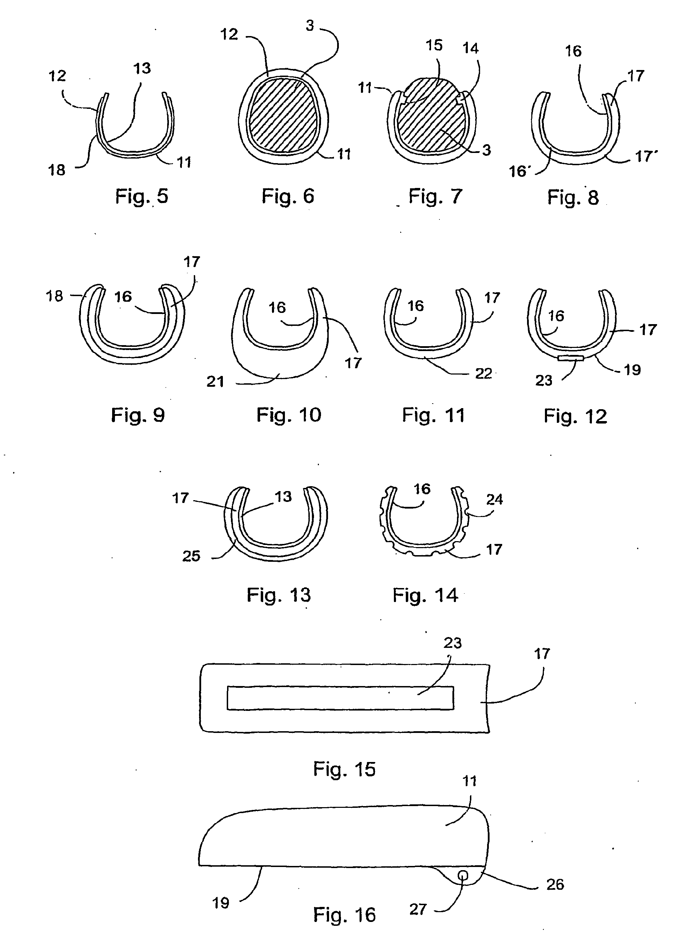

The handle section 3 is encircled by an endless rib or ridge 9 defining a kind of seat and the ridge is integrated in the casing. However, in one presented example (cf Fig. 6) the ridge 9 is missing.

In order to customize the injection device to the user's or owner's personal requirements, needs and to provide vital information to medical personnel regarding the state of health or treatment of the user an element 11 has been invented, which is intended to be removable placed over the handle section 3 by the user of an ordinary portable injection device, eg the pen-type injector of Fig. 1. This personal element 11 is an essentially shell-formed element having a curved inner face 12 possessing a configuration substantially conforming to the shape of the handle section or said seat and has a length corresponding thereto. Thus, when mounted said element 11 preferably occupies the whole seat (if any) and works as a grip for the injection device. In cross section said element 11 is substantially U-shaped and is clamped over the handle section, cf Figs. 2 and 5. As shown in Fig. 5 said cross section is more than semi circular and less than a full circle. This means that, when applying the element 11 on the handle section, the legs of the "U" is resiliently forced apart and then they will spring back and clamp fastened to the handle section 3. If the owner of the injection device wants to substitute the element 11 he just has to bend it lose, remove it and put an other, preferred element in its.place, cf the substitute element 11' depicted in Fig. 1, by pushing it sidways over the handle section 3. If, for any reason, the owner wants to keep the originally chosen element he may of course fasten it by means of an adhesive.

Referring now to Fig. 5 the element 11 preferably has a friction enhancing structure 13, eg grooves, small protrusions with sharp edges, particulate material integrated in the inner face 12 and so on, to eliminate the chance of the applied element sliding on the slippery surface of the handle section 3. Alternately or in addition to said structure the inner face 12 may be provided with a friction enhancing coating, e.g. a layer of rubber-based material

An other way of fixing the element 11 on the handle section 3 is by a detent-recess lock. An example of said fixation is schematically illustrated in Fig. 7. A number of detents or small pins 14 protrude from the inner face 12 of the element 11 to be snapped into correspondingly shaped, interengaging recesses or holes 15 in the handle section.

Referring to Figs. 8 and 9 the element 11 according to present invention alternately is composed of two or more materials permanently joined in the axial direction and/or the radial direction of said element. Figs. 8 and 9 are cross section views showing two examples thereof. When the element has more than one layer the innermost layer 16 is of a more pliable material than that of the handle section 3 but less than that of a superimposed layer 17. As the innermost layer 16 works as an anchoring means it is made of resilient material and is rather thin. The superimposed layer 17 of elastic material is the actual grip portion and is softer and thicker than the layer 16 to give the user of the injection device a feeling of comfort and a steady holding of the injection device, when used. Preferably, the superimposed layer is a material possessing low thermal conductivity giving the user a pleasant feeling of warmth when handling the injection device. Fig 9 illustrates that, according to one embodiment of the invention, a friction enhancing coating or layer 18 encloses the element 11. This layer 18 is thinner than layers 16 and 17 to be easily deformed and flexibly accompany any depression of the thicker layer 16 when the user squeezes the element 11. Alternately or in addition to said layer 18 the outer face of the element 11 possesses a friction enhancing structure similar to the one discussed above in conjunction with Fig. 5. Actually, all embodiments of the element of present invention preferably has an outer face made non-slippery, cf Fig. 2.

In order to better adapt the element 11 to the configuration of the hand of the user of the injection device or to his handling ability or wishes the soft grip layer 17 may be provided with portion(-s) 21 of a thickness larger than the general thickness of the element 11 and/or with portion(-s) 22 of a thickness smaller than the general thickness of the element, cf Fig. 10 and 11, respectively. Preferably the element is given a contour matching the palm and fingers of the specific hand with which the user handles the injection device, cf Fig. 4. Said contour is preferably pre-made but could of course be configured afterwards.

To make it possible to distinguish injection devices of the same brand from each other the elements according to present invention are envisaged to be available with an outer face 19 having a specific pattern 28 and/or colour(-s) chosen by the owner of the device, an example of which is presented in Fig. 3. When the owner wants to give the device a new and different appearance he has only to substitute said element.

Instead of or as a complement to the specific pattern 28 provided on the outer face 19 a sign, a bar-code, braille or characters are suitable incorporated in the surface. Hereby it is possible to display information regarding the owner of the injection device and/or dosing instructions to the user. Thus, a lost device is identified by the name and address printed on the outer face of the element. Of still more importance is the opportunity to provide the device with vital medical instructions, such as kind of drug administrated by the injection device, dosing instructions, warning sign etc. This information would be very helpful for a medical attendant who is to treat an unconscious injection device user and could be a question of life and death.

An other way to give personal information about the owner and/or his medical needs is to produce the element 11 of a translucent or transparent material 17' and position a paper, sheeting film or the like 16', having said information and/or pattern, between the handle section 3 and the element 11, whereby the information is visible through the translucent layer 17' (cf Fig. 8).

The element 11 according to present invention even offers the opportunity to incorporate a chosen metering instrument in accordance with the owner's wishes or needs, such as a liquid crystal thermometer denoting the temperature of the drug ampoule, a blood sugar meter enabling the user to test himself and/or other benefical meters. Some persons would like to have a digital watch or a magnetic tape with recorded information attached to the device. As an example of this Figs. 12 and 15 show an element 11 according to present invention having a thermometer 23 integrated in said superimposed layer 17 and in level with said outer face 19.

It is also possible to give the injection device a specific (parfume) scent by enclosing micro-ampoules 24 containing an aromatic substance in the outer layer, eg said superimposed layer 17, as illustrated in Fig. 14.

Some persons appreciate a warm grip on an injection device and Fig. 13 depicts a heat reflective sheet 25 enclosed in the outer layer 17 adjacent to or at the outer face 19 of the element 11.

It is also popular among some groups of persons to have a personal item dangling from a belonging. This demand is fulfilled according to present invention by a ring-shaped mount, an eye, a protrusion with a through hole, a clamp or a similar device which is firmly attached to or integrated in said element for optional fastening of a string, a thread, a chain etc carrying a charm, a mascot or the like (not shown).

Thus, Fig. 16 shows a protrusion 26 with a through hole 27 arranged on the outer face 19 of the element 11. A similar attachment can alternatively be arranged on the housing itself, e.g. at the rear not to interfere with handling of the device.

Preferably said protecting cap 8 of the injection device has an outer face provided with a pattern, one or more colours, characters, a sign or the like matching that of the outer face of the element 11.

A variety of embodiments of present invention have been exemplified in the description and the drawings and the features presented therein may of course be optionally combined in the element of present invention according to the requirements of the user of the injection device.

## Claims

1. An injection device suitable for modification, said injection device comprising:
a) a housing (1-3) designed or suitable for manual gripping at a handle (3),
b) a container for a preparation attachable to (6) or enclosed in the housing,
c) an outlet (4) for the preparation exposed with respect to the housing,
d) a mechanism arranged for moving the preparation at least from the container through the outlet, **characterised in that** the device further comprises
e) a set of elements (11, 11'), wherein the elements are different in at least one respect or property and wherein each element has an outer face (19) possessing a structure, configuration and/or a function fulfilling personal requirements of the user and that the housing and the element are mutually adapted to give a releasable attachment,
and further **characterised in that**
the handle has a ridge (9) forming a site or seat to which is attachable a first one of the set of elements (11, 11'), and
**in that** each element (11, 11') is U-shaped in cross-section, having a curved inner face (12) possessing a configuration substantially conforming to the shape of the handle, said element being adapted to be brought into releasable clamping engagement with the handle (3) in the site or seat (9), and that each element (11,11') has a length essentially corresponding to the length of said handle (3) and serving as a grip for the injection device.

2. The injection device according to claim 1 with a second element different from a first one in at least one respect selected from the group consisting of information carried, aesthetic properties, functional properties, size and/or shape or combinations thereof.

3. The injection device according to claim 2, wherein the inner face (12) of at least one element (11) has a friction enhancing structure (13) and/or a friction enhancing coating.

4. The injection device according to any of claims 2 or 3, wherein at least one element (11) has protrusions (14) projecting inwardly from its inner face (12) and arranged to fixingly engage with complementary shaped recesses (15) in the handle (3) of the injection device.

5. The injection device according to any of claims 2 to 4, wherein at least one element (11) comprises one or more material (s) (16-18) more pliable than the material of the handle (3).

6. The injection device according to claim 5, wherein said element (11) includes an inner layer (16) of a first resilient material and a superimposed layer (17) of a second elastic material softer than said first material.

7. The injection device according to claim 5 or 6, wherein said element (11) comprises an outer layer (17) having a outer face possessing a friction enhancing structure and/or a friction enhancing coating (18).

8. The injection device according to claim 6 or 7, wherein at least two of the layers (16, 17) of said element (11) are of mutually different thicknesses.

9. The injection device according to any of claims 2 to 8, wherein at least one element (11) includes at least one portion (21) having a thickness substantially larger that the general thickness of said element.

10. The injection device according to any of claims 2 to 9, wherein at least one element (11) includes at least one portion (22) having a thickness substantially smaller than the general thickness of said element.

11. The injection device according to claims 9 and 10, wherein said element has a contour (17, 21, 22) matching the palm and the fingers of the user.

12. The injection device according to any of claims 2 to 11, wherein the outer face (19) of at least one element (11) is provided with a pattern (21), one or more colours, characters, a sign or the like.

13. The injection device according to any of claims 2 to 12, wherein at least one element (11') is translucent, whereby a paper, sheeting, film or the like (16') provided with a pattern, characters, a sign or information positioned between said handle section (3) and the element (11') is visible therethrough.

14. The injection device according to claim 12, wherein the outer face (19) of said element (11) is provided with the name of the user, dosing instructions, Braille and/or a bar code.

15. The injection device according to claim 13 or 14, wherein the outer face (19) of said element (11) is painted with a fluorescent dye.

16. The injection device according to any of claims 2 to 15, wherein a magnetic tape, a liquid crystal thermometer (23), a blood sugar meter and/or a digital watch is (are) integrated in the outer layer (17) of at least one element (11).

17. The injection device according to any of claims 2 to 16, wherein micro-ampoules (24) containing an aromatic substance are arranged in the outer layer (17) of at least one element (11).

18. The injection device according to any of claims 2 to 17, wherein a heat reflective sheet (25) is enclosed in the outer layer (17) of at least one element (11).

19. The injection device according to any of claims 2 to 18, wherein a ring-shaped mount, an eye, a protrusion (26) with a through-hole (27), a clamp or a similar device is firmly attached to or integrated in at least one element for optional fastening of a string, a thread, a chain etc carrying a charm, a mascot or the like.

20. The injection device according to any of claims 2 to 19, further including a protecting cap (8) with an outer face provided with a pattern, one or more colours, characters, a sign or the like matching that of the outer face of said element.

## Patentansprüche

1. Injektionsvorrichtung, die zur Modifikation geeignet ist, wobei die Injektionsvorrichtung umfasst:
a) ein Gehäuse (1-3), das zum manuellen Greifen an einem Haltegriff (3) konzipiert oder geeignet ist,
b) einen Behälter für eine Zubereitung befestigbar an (6) oder eingeschlossen in dem Gehäuse,
c) einen Auslass (4) für die Zubereitung, der bezüglich des Gehäuses freiliegt,
d) einen Mechanismus, der zur Bewegung der Zubereitung wenigstens aus dem Behälter durch den Auslass angeordnet ist, **dadurch gekennzeichnet, dass** die Vorrichtung außerdem umfasst:
e) einen Satz von Elementen (11, 11') wobei die Elemente in wenigstens einer Hinsicht oder Eigenschaft unterschiedlich sind, wobei jedes Element eine Außenseite (19) hat, die eine Struktur, eine Konfiguration und/oder eine Funktion besitzt, die persönliche Anforderungen des Verwenders erfüllt und dass das Gehäuse und das Element wechselseitig angepasst sind, um eine lösbare Befestigung zu ergeben,
und außerdem **dadurch gekennzeichnet, dass**
der Haltegriff einen Wulst (9) hat, der eine Stelle oder einen Sitz bildet, an dem ein erstes des Satzes von Elementen (11, 11') befestigbar ist, und
**dadurch**, dass jedes Element (11, 11') im Querschnitt U-förmig ist, wobei es eine gebogene Innenseite (12) hat, die eine Konfiguration besitzt, die sich im Wesentlichen an die Form des Haltegriffes anpasst, wobei das Element angepasst ist, um in lösbaren Klemmeingriff mit dem Haltegriff (3) an der Stelle oder dem Sitz (9) gebracht zu werden, und dass jedes Element (11, 11') eine Länge hat, die im Wesentlichen der Länge des Haltegriffs (3) entspricht, und als Griff für die Injektionsvorrichtung dient.

2. Injektionsvorrichtung gemäß Anspruch 1, wobei ein zweites Element sich von einem ersten in wenigstens einer Hinsicht unterscheidet, ausgewählt aus der Gruppe, bestehend aus getragener Information, ästhetischen Eigenschaften, funktionellen Eigenschaften, Größe und/oder Gestalt oder Kombinationen davon.

3. Injektionsvorrichtung gemäß Anspruch 2, wobei die Innenseite (12) wenigstens eines Elements (11) eine die Reibung verstärkende Struktur (13) und/oder eine die Reibung verstärkende Beschichtung hat.

4. Injektionsvorrichtung gemäß einem der Ansprüche 2 und 3, wobei wenigstens ein Element (11) Vorsprünge (14) hat, die aus seiner Innenseite (12) nach innen vorstehen und angeordnet sind, um mit komplementär geformten Aussparungen (15) im Haltegriff (3) der Injektionsvorrichtung fixierend in Eingriff zu gelangen.

5. Injektionsvorrichtung gemäß einem der Ansprüche 2 bis 4, wobei wenigstens ein Element (11) ein Material oder mehrere Materialien (16-18) umfasst, das/die flexibler ist/sind als das Material des Haltegriffs (3).

6. Injektionsvorrichtung gemäß Anspruch 5, wobei das Element (11) eine innere Schicht (16) eines ersten elastischen Materials und eine darauf angeordnete Schicht (17) eines zweiten elastischen Materials, das weicher als das erste Material ist, umfasst.

7. Injektionsvorrichtung gemäß Anspruch 5 oder 6, wobei das Element (11) eine äußere Schicht (17) umfasst, die eine Außenseite hat, die eine die Reibung verstärkende Struktur und/oder eine die Reibung verstärkende Beschichtung (18) besitzt.

8. Injektionsvorrichtung gemäß Anspruch 6 oder 7, wobei wenigstens zwei der Schichten (16, 17) des Elements (11) wechselseitig unterschiedliche Dicken haben.

9. Injektionsvorrichtung gemäß einem der Ansprüche 2 bis 8, wobei wenigstens ein Element (11) wenigstens einen Teil (21), der eine Dicke hat, die wesentlich größer ist als die allgemeine Dicke des Elements, umfasst.

10. Injektionsvorrichtung gemäß einem der Ansprüche 2 bis 9, wobei wenigstens ein Element (11) wenigstens einen Teil (22), der eine Dicke hat, die wesentlich kleiner als die allgemeine Dicke des Elements ist, umfasst.

11. Injektionsvorrichtung gemäß den Ansprüchen 9 und 10, wobei das Element eine Kontur (17, 21, 22) hat, die zu der Handfläche und den Fingern des Benutzers passt.

12. Injektionsvorrichtung gemäß einem der Ansprüche 2 bis 11, wobei die Außenseite (19) wenigstens eines Elements (11) mit einem Muster (21), einer oder mehreren Farben, Schriftzeichen, einem Symbol oder dergleichen versehen ist.

13. Injektionsvorrichtung gemäß einem der Ansprüche 2 bis 12, wobei wenigstens ein Element (11') durchscheinend ist, wodurch ein Papier, eine Folie, ein Film oder dergleichen (16'), versehen mit einem Muster, Schriftzeichen, einem Symbol oder Information, positioniert zwischen dem Haltegriffabschnitt (3) und dem Element (11'), **dadurch** sichtbar ist.

14. Injektionsvorrichtung gemäß Anspruch 12, wobei die Außenseite (19) des Elements (11) mit dem Namen des Benutzers, Dosierungsinstruktionen, Blindenschrift und/oder einem Strichcode versehen ist.

15. Injektionsvorrichtung gemäß Anspruch 13 oder 14, wobei die Außenseite (19) des Elements (11) mit einem Fluoreszenzfarbstoff angestrichen ist.

16. Injektionsvorrichtung gemäß einem der Ansprüche 2 bis 15, wobei ein Magnetstreifen, ein Flüssigkristallthermometer (23), ein Blutzuckermessgerät und/oder eine digitale Uhr in der äußeren Schicht (17) wenigstens eines Elements (11) integriert ist/sind.

17. Injektionsvorrichtung gemäß einem der Ansprüche 2 bis 16, wobei Mikroampullen (24), die eine aromatische Substanz enthalten, in der äußeren Schicht (17) wenigstens eines Elements (11) angeordnet sind.

18. Injektionsvorrichtung gemäß einem der Ansprüche 2 bis 17, wobei eine Wärme reflektierende Folie (25) in der äußeren Schicht (17) wenigstens eines Elements (11) eingeschlossen ist.

19. Injektionsvorrichtung gemäß einem der Ansprüche 2 bis 18, wobei ein ringförmiger Rahmen, ein Auge, ein Vorsprung (26) mit einem Durchgangsloch (27), eine Klammer oder eine ähnliche Vorrichtung fest mit wenigstens einem Element verbunden ist oder in wenigstens einem Element integriert ist zur optionalen Befestigung einer Schnur, eines Fadens, einer Kette usw. der/die einen Talisman, ein Maskottchen oder dergleichen trägt.

20. Injektionsvorrichtung gemäß einem der Ansprüche 2 bis 19, außerdem umfassend eine Schutzkappe (8) mit einer Außenseite, die mit einem Muster, einer oder mehreren Farben, Schriftzeichen, einem Symbol oder dergleichen versehen ist, passend zu der Außenseite des Elements.

## Revendications

1. Dispositif d'injection pouvant subir des modifications, ledit dispositif d'injection comprenant :
a) un logement (1-3) conçu pour, ou adapté à, la préhension manuelle au niveau d'une poignée (3),
b) un récipient pour une préparation (6), fixable sur, ou contenu dans, le logement,
c) une sortie (4) pour la préparation, à accès direct par rapport au logement,
d) un mécanisme conçu pour déplacer la préparation au moins depuis le récipient jusqu'à travers la sortie, **caractérisé en ce que** le dispositif comprend en outre :
e) un jeu d'éléments (11, 11') dans lequel les éléments diffèrent entre eux à au moins un égard ou pour au moins une propriété et dans lequel chaque élément a une face extérieure (19) possédant une structure, une configuration et/ou une fonction satisfaisant les besoins personnels de l'utilisateur, le logement et l'élément étant mutuellement adaptés pour donner une fixation réattachable,
ledit dispositif étant en outre **caractérisé :**
**en ce que** la poignée a une arête (9) formant un site ou un siège sur lequel peut se fixer un premier élément du jeu d'éléments (11, 11'), et
**en ce que** chaque élément (11, 11') est en forme de U en section transversale et a une face interne incurvée (12) possédant une configuration épousant sensiblement la forme de la poignée, ledit élément étant adapté à venir en prise libérable, par pinçage, avec la poignée (3) au niveau du site ou du siège (9), et en ce que chaque élément a une longueur correspondant essentiellement à la longueur de ladite poignée (3) et servant de point d'ancrage pour le dispositif d'injection.

2. Dispositif d'injection selon la revendication 1, ayant un second élément différent du premier à au moins un égard, sélectionné dans le groupe consistant en l'information indiquée, les propriétés esthétiques, les propriétés fonctionnelles, la taille et/ou la forme, ou des combinaisons de ces caractéristiques.

3. Dispositif d'injection selon la revendication 2, dans lequel la face interne (12) d'au moins un élément (11) a une structure augmentant la friction (13) et/ou un revêtement augmentant la friction.

4. Dispositif d'injection selon l'une quelconque des revendications 2 ou 3, dans lequel au moins un élément (11) a des protubérances (14) se projetant intérieurement depuis sa face interne (12) et conçu pour venir en prise de fixation avec des creux de forme complémentaire (15) ménagés dans la poignée (3) du dispositif d'injection.

5. Dispositif d'injection selon l'une quelconque des revendications 2 à 4, dans lequel au moins un élément (11) comprend un ou plusieurs matériau(x) (16-18) plus flexible(s) que le matériau de la poignée (3).

6. Dispositif d'injection selon la revendication 5, dans lequel ledit élément (11) inclut une couche interne (16) composée d'un premier matériau résilient et une couche superposée (17) composée d'un second matériau élastique plus souple que ledit premier matériau.

7. Dispositif d'injection selon la revendication 5 ou 6, dans lequel ledit élément (11) comprend une couche externe (17) ayant une face extérieure possédant une structure augmentant la friction et/ou un revêtement augmentant la friction (18).

8. Dispositif d'injection selon la revendication 6 ou 7, dans lequel au moins deux des couches (16, 17) dudit élément (11) sont d'épaisseur différente l'une par rapport à l'autre.

9. Dispositif d'injection selon l'une quelconque des revendications 2 à 8, dans lequel au moins un élément (11) inclut au moins une portion (21) ayant une épaisseur sensiblement supérieure à l'épaisseur générale dudit élément.

10. Dispositif d'injection selon l'une quelconque des revendications 2 à 9, dans lequel au moins un élément (11) inclut au moins une portion (22) ayant une épaisseur sensiblement inférieure à l'épaisseur générale dudit élément.

11. Dispositif d'injection selon les revendications 9 et 10, dans lequel ledit élément (11) a un contour (17, 21, 22) épousant la paume et les doigts de l'utilisateur.

12. Dispositif d'injection selon l'une quelconque des revendications 2 à 11, dans lequel la face extérieure (19) d'au moins un élément (11) comporte un motif (21), une ou plusieurs couleurs, un ou plusieurs caractères, un signe, ou analogues.

13. Dispositif d'injection selon l'une quelconque des revendications 2 à 12, dans lequel au moins un élément (11') est translucide, grâce à quoi un papier, une feuille, un film ou analogue (16') possédant un motif, des caractères, un signe ou une information, positionné(e) entre ladite section de la poignée (3) et l'élément (11'), est visible au travers de ce dernier.

14. Dispositif d'injection selon la revendication 12, dans lequel la face extérieure (19) dudit élément (11) comporte le nom de l'utilisateur, les instructions de dosage, du braille et/ou un code barre.

15. Dispositif d'injection selon la revendication 13 ou 14, dans lequel la face extérieure (19) dudit élément (11) est peinte avec un colorant fluorescent.

16. Dispositif d'injection selon l'une quelconque des revendications 2 à 15, dans lequel une bande magnétique, un thermomètre à cristaux liquides (23), un appareil mesurant le glucose sanguin et/ou une montre à affichage numérique est (sont) intégré(e)(s) dans la couche externe (17) d'au moins un élément (11).

17. Dispositif d'injection selon l'une quelconque des revendications 2 à 16, dans lequel des micro-ampoules (24) contenant une substance aromatique sont disposées dans la couche externe (17) d'au moins un élément (11).

18. Dispositif d'injection selon l'une quelconque des revendications 2 à 17, dans lequel une feuille réflectrice de chaleur (25) est contenue dans la couche externe (17) d'au moins un élément (11).

19. Dispositif d'injection selon l'une quelconque des revendications 2 à 18, dans lequel un support annulaire, un oeilleton, une protubérance (26) ayant un trou traversant (27), une pince ou un dispositif similaire est fermement fixé à, ou intégré dans, au moins un élément pour permettre, facultativement, la fixation d'un cordon, d'un fil, d'une chaîne, etc. comportant une breloque, une mascotte, ou analogue.

20. Dispositif d'injection selon l'une quelconque des revendications 2 à 19, incluant en outre un capuchon protecteur (8) ayant une face extérieure comportant un motif, une ou plusieurs couleurs, un ou plusieurs caractères, un signe, ou analogues, correspondant à celui(ceux)/celle(s) de la face extérieure dudit élément.
